# EUROPEAN PATENT APPLICATION

(11) **EP 2 639 314 A1**
(43) Date of publication of application: **18.09.2013**
(21) Application number: 12159546.6
(22) Date of filing: 14.03.2012
(51) Int. Cl.: C12Q 1/68

(54) **In vitro method for predicting whether a compound is genotoxic in vivo.**

(71) Applicant: Universiteit Maastricht, 6211 LK Maastricht (NL); Academisch Ziekenhuis Maastricht, 6229 HX Maastricht (NL)
(72) Inventor: Lizarraga-Lopez, Daneida, 6221 JG Maastricht (NL); Rieswijk, Linda, 6227 RP Maastricht (NL); Van Delft, Joseph Henri Marie, 1040 Etterbeek (BE); Kleinjans, Joseph Catharina Stephanus, 6221 AR Maastricht (NL)
(74) Representative: Habets, Winand

(57) **Abstract**

The invention is in the field of genomics and it provides an in vitro method for predicting whether a compound is genotoxic in vivo. It provides a method that employs the analysis of expression profiles of microRNAs in cells exposed to a potentially genotoxic compound. It was found that differential expression of a number of microRNAs could reliably predict whether a compound was a genotoxic or a non-genotoxic compound.

SEQUENCE LISTING

<110>  Universiteit Maastricht
       Academisch Ziekenhuis Maastricht

<120>  IN VITRO METHOD FOR PREDICTING WHETHER A COMPOUND IS GENOTOXIC IN
        VIVO.
<130>  213 EPP0
<160>  10
<170>  PatentIn version 3.5

<210>  1
<211>  22
<212>  RNA
<213>  homo sapiens
<400>  1
aggcagcggg guguagugga ua                                              22

<210>  2
<211>  22
<212>  RNA
<213>  homo sapiens
<400>  2
gacugacacc ucuuugggug aa                                              22

<210>  3
<211>  22
<212>  RNA
<213>  homo sapiens
<400>  3
ugagguagua aguuguauug uu                                              22

<210>  4
<211>  22
<212>  RNA
<213>  homo sapiens
<400>  4
augggugaau uuguagaagg au                                              22

<210>  5

<211>  23
<212>  RNA
<213>  homo sapiens
<400>  5
cucucaccac ugcccuccca cag                                                23

<210>  6
<211>  22
<212>  RNA
<213>  homo sapiens
<400>  6
cugggagagg guuguuuacu cc                                                 22

<210>  7
<211>  22
<212>  RNA
<213>  homo sapiens
<400>  7
ugcaacuuac cugagucauu ga                                                 22

<210>  8
<211>  22
<212>  RNA
<213>  homo sapiens
<400>  8
acuuuaacau ggaagugcuu uc                                                 22

<210>  9
<211>  19
<212>  RNA
<213>  homo sapiens
<400>  9
aauggauuuu uggagcagg                                                     19

<210>  10
<211>  22
<212>  RNA
<213>  homo sapiens
<400>  10
aaugcaccug ggcaaggauu ca                                                 22

## Description

### Field of the invention

The invention is in the field of genomics and it provides an in vitro method for predicting whether a compound is genotoxic in vivo.

### Background of the invention

The classic 2 year rodent bioassay is the standard test for identifying the carcinogenic potential of chemical compounds. Such tests are time-consuming and costly. Moreover, they require the sacrifice of many animal lives. In vitro systems are therefore preferred; however, there is no reliable in vitro method for accurately predicting the genotoxicity of a compound in vivo.(1,2).

Well-established in vitro systems frequently used to identify the genotoxic potential of chemical compounds are for instance the bacterial Ames test, the mouse lymphoma assay, the micronucleus test and the chromosomal aberration test (3).

These classic in vitro genotoxicity tests, however, have been shown to generate an extremely high false positive rate when compared to in vivo carcinogenicity data. (3). False positive in this context means that the compound yields a positive result in the in vitro assay whereas it is negative for genotoxicity in an in vivo assay.

Because of the low predictive value of current in vitro assays, a compound that tested positive in an in vitro assay has to be retested in an in vivo assay in order to verify whether the compound is a true genotoxic (GTX) compound. This generates a lot of extra costs and efforts, as well as the sacrifice of many animal lives.

Therefore, new and more predictive in vitro systems are desired in the art which are capable of reliably discriminating genotoxins from non-genotoxins.

### Summary of the invention

The present invention employs the analysis of expression profiles of microRNAs in human hepatocellular carcinoma cells under the influence of potentially genotoxic compounds. In that way we were able to discriminate true in vivo GTX compounds from non-GTX compounds (NGTX).

Hence, the invention relates to an in vitro method for distinguishing between genotoxic and non-genotoxic compounds by determining the expression level of at least microRNA 1262 and comparing the expression level thus obtained with a reference value of expression of microRNA 1262 wherein it is concluded that a compound is genotoxic if the expression of said microRNA 1262 is below the reference value.

### Detailed description of the invention

Chemical compounds, which are able to cause gene mutations or chromosomal damage in vivo are herein defined as true genotoxins (true GTX) (6, 12). False positive genotoxins (false positive GTX or false GTX) are herein defined as compounds that are not capable of causing gene mutations or chromosomal damage in vivo, but are positive in a conventional in vitro assay for genotoxicity.

Gene mutations or chromosomal damage may occur when the compound covalently binds to DNA in vivo. Such binding to DNA may be not or incorrectly repaired which may lead to mutations accumulating in time and ultimately inducing the formation of tumors (6, 12).

The present invention employs the analysis of expression profiles of microRNA 1262 as an in vitro system to discriminate GTX compounds from non-GTX compounds.

It was found that differential expression of microRNA 1262 could reliably predict whether a compound was a true genotoxic compound. So as a first step in the method according to the invention, a cell or cell line is provided, preferably a human cell line, even more preferably a human hepatocellular carcinoma cell line. Most preferred is a HepG2 cell line (ATCC HB-6065).

The skilled person is aware of the various methods that may be used to obtain a suitable cell or cell line. The examples provided herein may provide additional guidance.

In a further step of the method according to the invention, an assay is provided capable of determining the expression level of microRNA 1262 (miR 1262).

Assays that may determine expression of miR 1262 are also known in the art. Such an assay may consist of a single PCR- based assay or a hybridization assay. In the alternative, a multiplex assay may be used, consisting of a plurality of different assays that can be performed simultaneously. This allows for the determination of simultaneous expression of more than one gene. Even more advantageously, the assay is a nucleic acid microarray such as a DNA microarray, such as a GeneChip ® provided by Affymetrix. Preferrred is the use of a miRCURY™ locked-nuclei acid array (LNA)array as provided by Exiqon, Denmark.

In another step of the method according to the invention, the compound to be tested for genotoxicity is contacted with the cell line. The time of contact may differ somewhat depending on the particular cell line chosen and should be determined empirically. For the HepG2 cell line, 12 to 24 hours appeared a suitable time, this is however not critical. The skilled person will be aware of the metes and bounds of this step.

In the examples section, the concentrations used for 3 GTX compounds and 6 non-GTX (NGTX) compounds are provided as guidance. In general, the use of cytotoxic concentrations should be avoided. The skilled person will know how to avoid using cytotoxic concentrations of test compounds.

In order to obtain reproducible results, it was found advantageous to obtain at least three independent readings of the gene expression. Hence, the above steps may be repeated at least twice to obtain a more reproducible and reliable result.

Hence, the invention relates to an in vitro method for determining whether a compound is genotoxic or non-genotoxic compounds said method comprising the steps of:
a. exposing a cell to the compound for a certain amount of time,
b. determining the expression level of at least one microRNA marker in said cell,
c. comparing said expression level with a predetermined reference value and determining whether the expression level of the at least one microRNA marker is below or above the reference value,
d. determining whether the compound is a genotoxic compound or a non-genotoxic compound
wherein the at least one microRNA marker is microRNA 1262.

It appeared that the method could be improved as described herein below by measuring the expression levels of at least three microRNAs. In a preferred embodiment, the invention therefore relates to an in vitro method as described above wherein the at least one microRNA is microRNA 1262, microRNA 98 and microRNA 30c-1.

The method could be further improved by employing the expression levels of ten microRNAs. In a further preferred embodiment, the invention relates to an in vitro method as described above wherein the at least one microRNA is microRNA 1262, microRNA 98, microRNA 30c-1, microRNA 885-3p, microRNA 888*, microRNA 1229, microRNA 891b, microRNA302b, microRNA 1246 and microRNA 502-3p.

It is preferred to use a HepG2 cell line in the above invention, the preferred incubation times for the compound is in the order of 12 - 24 hours.

Particularly good results were obtained when the above method steps a - d are performed at least in triplicate thereby obtaining expression levels at least in triplicate and wherein the step of determining whether the compound is a genotoxic compound or a non-genotoxic compound is based on the average of such at least in triplicate expression levels.

### Examples

### Example 1: Cells and culture.

Human hepatocellular carcinoma HepG2 cells (ATCC HB-6065) were used in all the experiments. HepG2 cells were maintained as a monolayer culture in 95% humidity, atmosphere with 5% of CO2 and 37 °C. HepG2 cells were passaged at pre-confluent densities with trypsin-EDTA solution. Cells were cultured and passaged in Minimal Essential Medium (MEM) supplemented with 10% of Foetal Bovine serum, 1% penicillin/streptomycin, 1% sodium-pryruvate and 1% non-essential amino acids. All media compounds were obtained from Gibco BRL (Breda, the Netherlans). Two milliliters of cells (7x10^4/mL) were seeded into each well in a 6-wells microtiter plate. HepG2 cells were exposed to 9 compounds at IC20-72hrs values as specified in Table 1 and to a vehicle control (0.5% DMSO and PBS) during 12 hours. All measurements were performed in triplicate.

### Example 2: RNA isolation

Total RNA was isolated after 24 hours of incubation with the nine compounds (AFB1, BaP, Cispl, 8HQ, Que, E2, AmpC, CsA, TCDD) or controls (DMSO and PBS). Isolation of total RNA was done by using miRNeasy mini Kit (Qiagen Westburg bv, Leusden, the Netherlands) according to the manufacturer's instructions and followed by a DNAse I (Qiagen Inc.) treatment. RNA quantity was measured on a spectrophotometer and quality was determined by BioAnalyzer (Agilent Technologies, Breda, the Netherlands). Only RNA samples which showed clear 18S and 28S peaks and with a RIN level higher than 8 were used.

### Example 3: Determining MicroRNA expression using Exiqon arrays.

The miRCURY™ locked-nuclei acid array (LNA), 5th generation (Exiqon, Denmark) contains probes that detect mature forms of all microRNAs present in miRBase 15.0 (http://www.mirbase.org/). The Exiqon platform has been validated and is shown to reliably detect microRNA expression. The microarray contains 9360 reporters, each present four times on the microarray. In our analysis 1326 human and viral reporters were used, of which 880 are unique human mature microRNAs. The other 8024 reporters represent microRNAs from other species. cDNA was generated using 1 μg of total RNA per sample. RNA was labeled only with Hy3 containing dye using the mercury LNA™ microRNA Hy3 Power labeling kit (Exiqon, Denmark) according to the manufacturer's protocol in a total volume of 25 μl. To adjust the volume to 100 μl, a hybridization buffer provided in miRCURY LNA™ microRNA Array, 5th generation kit (Exiqon, Denmark) was used. In a total volume of 100 μl the sample was incubated for 5 minutes at 95oC, spinned for 2 minutes at maximal speed and injected in the hybridization station. Hybridization was performed in a Tecan HS4800 Pro Hybridization station according to manufacturer protocol.

**Table 1: Compounds used in the study**

| **Compound** | **Abbreviation** | **CAS no** | **Dose** | **Solvent** | **Ames test result** | **GTX in vivo** |
|---|---|---|---|---|---|---|
| Aflatoxin B1 | AFB1 | 1162-65-8 | 1 μM | DMSO | + | + |
| Benzo[a]pyere | BaP | 50-32-8 | 2 μM | DMSO | + | + |
| Cisplatin | CisPl | 15663-27-1 | 7uM | PBS | + | + |
| 8-quinolinol | 8HQ | 148-24-3 | 15 μM | DMSO | + | - |
| Quercetin | Que | 117-39-5 | 50 μM | DMSO | + | - |
| 17beta-estradiol | E2 | 50-28-2 | 30μM | DMSO | - | - |
| Ampicillin trihydrate | AmpC | 7177-48-2 | 250μM | DMSO | - | - |
| Cyclosporine A | CsA | 59865-13-3 | 3μM | DMSO | - | - |
| 2,3,7,8-tetrachloro dibenzo-p-dioxin | TCDD | 1746-01-6 | 10nM | DMSO | - | - |

For each biological experiment, one hybridization per time point was conducted and one sample per array. After hybridization, the microarray slides were washed accordingly Exiqon instructions. Then arrays were scanned using the GenePix 4000A scanner (Axon Instruments, Foster City, CA). To quantify the signals, the images were processed to TXT files through the GenePix Pro Sofware Suite version 3.0 by using the GAL file for probe annotation as obtained from Exiqon.

The GAL file is based on miRBase version 15.0. These GPR files were then imported in R 2.11.0, for quality control and statistical analysis. R package is freely available for academic use from the Comprehensive R Archive Network (http://www.cran.r-project.org/).

First, the quality of all arrays was inspected using arrayQC, an in-house quality control pipeline that generates virtual images, boxplots, correlation plots, clustering images, MvA and PCA plots. Spike-ins were used for all arrays as an extra quality check. Within this dataset, no arrays were technically deviating.

During data analysis the spot intensities were background-corrected and then filtered for low signals (i.e. intensity < 10). Only the viral and human microRNA reporter intensities (1.326 unique reporters x 4 technical replicates) were used further. If for a reporter less than 3 of the 4 technical replicates were left, then the reporter intensity on that specific reporter was set to NA. The data were log2 transformed, followed by a quantile normalization and a summarization step. In the summarization step, the median intensity from the remaining technical replicates per condition was calculated. Finally, for each reporter a cut-off filtering was applied using the median of the three biological replicates. Only reporters that had a median intensity ≥50 for at least one condition of the 9 compounds tested, or their respective controls, passed this filtering per time point. A microRNA was considered as differentially expressed when the absolute Fold Change (FC) > 1.5 and the p-value for a paired t-test was < 0.1.

### Example 4: GTX or NGTX class prediction using microRNAs

In order to predict the class of a compound, average log2 ratios of expression values of 10 selected microRNAs were calculated for the GTX and NGTX compounds listed in Table 1. A compound was regarded as GTX or NGTX, when the microRNA was differentially expressed below or above a certain threshold value. Table 2 shows the threshold values applied herein for the 10 selected microRNAs. This was done for every microRNA separately. A compound was predicted to be in vivo GTX when more than 50% of the microRNAs gave the prediction in vivo GTX than in vivo NGTX. A compound was predicted to be in vivo NGTX when more than 50% of the microRNAs gave the prediction *in vivo* NGTX than *in vivo* GTX. If number of predictions for both classes were equal, the compound could not be assigned to a class.

**Table 2 microRNAs and their reference values for predicting in vivo genotoxicity**

| **miRNA_ID** | Reference Value | Prediction if Expression is above Reference value | Prediction if Expression is below Reference value |
|---|---|---|---|
| hsa-miR-885-3p | 0.407 | NGTX | GTX |
| hsa-miR-888* | -0.046 | GTX | NGTX |
| hsa-miR-98 | -0.043 | GTX | NGTX |
| hsa-miR-1262 | -0.060 | NGTX | GTX |
| hsa-miR-1229 | 0.224 | NGTX | GTX |
| hsa-miR-30c-1* | -0.093 | GTX | NGTX |
| hsa-miR-891b | 0.109 | GTX | NGTX |
| hsa-miR-302b | 0.096 | NGTX | GTX |
| hsa-miR-1246 | 0.554 | NGTX | GTX |
| hsa-miR-502-3p | -0.151 | GTX | NGTX |

Example 5: microRNA 1262 perfectly distinguishes GTX from NGTX compounds.

Prediction analysis with miR-1262 was conducted as described above using the reference value presented in Table 2. The results are shown in table 3.

**Table 3 Prediction analysis with microRNA 1262**

| | | | **miRNA 1262** | |
|---|---|---|---|---|
| **Compound** | **Abbreviation** | **GTX in vivo** | **Prediction** | **Score*** |
| Aflatoxin B1 | AFB1 | GTX | GTX | (2/3) |
| Benzo[a]pyere | BaP | GTX | GTX | (2/3) |
| Cisplatin | CisPl | GTX | GTX | (2/3) |
| 8-quinolinol | 8HQ | NGTX | NGTX | (2/3) |
| Quercetin | Que | NGTX | NGTX | (3/3) |
| 17beta-estradiol | E2 | NGTX | NGTX | (3/3) |
| Ampicillin trihydrate | AmpC | NGTX | NGTX | (2/3) |
| Cyclosporine A | CsA | NGTX | NGTX | (3/3) |
| 2,3,7,8-tetrachloro dibenzo-p-dioxin | TCDD | NGTX | NGTX | (3/3) |
| | | | 100% | 22/27 |
| Correctness of prediction | | | | (81%) |

| | | | | |
|---|---|---|---|---|
| * Score indicates the number of correct predictions of 3 independent experiments. | | | | |

MicroRNA 1262 scored each of the 9 compounds correctly, albeit that 5 out of 9 compounds were classified based on a score of 2 correct classifications out of three independent experiments. This resulted in a score of 81% when the score was averaged over 9 compounds.

### Example 6: Inclusion of other miRNAs in the analysis improves the correctness of prediction.

When the expression levels of microRNAs 98 and 30c-1 were included in the prediction analysis, the results improved. Notwithstanding the fact that microRNA 98 and 30c-1 on their own did not predict the genotoxicity of the compounds 100% correctly (tables 4 and 5), when their results were combined with those of microRNA 1262, their overall accuracy improved to 93% (Table 6).

**Table 4 Prediction analysis with microRNA 98**

| | | | **miRNA 98** | |
|---|---|---|---|---|
| **Compound** | **Abbreviation** | **GTX in vivo** | **Prediction** | **Score*** |
| Aflatoxin B1 | AFB1 | GTX | GTX | (2/3) |
| Benzo[a]pyere | BaP | GTX | GTX | (2/3) |
| Cisplatin | CisPl | GTX | GTX | (3/3) |
| 8-quinolinol | 8HQ | NGTX | NGTX | (3/3) |
| Quercetin | Que | NGTX | GTX | (1/3) |
| 17beta-estradiol | E2 | NGTX | NGTX | (2/3) |
| Ampicillin trihydrate | AmpC | NGTX | NGTX | (3/3) |
| Cyclosporine A | CsA | NGTX | NGTX | (3/3) |
| 2,3,7,8-tetrachloro dibenzo-p-dioxin | TCDD | NGTX | NGTX | (3/3) |
| | | | 89% | 22/27 |
| Correctness of prediction | | | | (81%) |

**Table 5 Prediction analysis with microRNA 30c-1**

| | | | **miRNA 30c-1** | |
|---|---|---|---|---|
| **Compound** | **Abbreviation** | **GTX in vivo** | **Prediction** | **Score*** |
| Aflatoxin B1 | AFB1 | GTX | GTX | (2/3) |
| Benzo[a]pyere | BaP | GTX | GTX | (2/3) |
| Cisplatin | CisPl | GTX | GTX | (2/3) |
| 8-quinolinol | 8HQ | NGTX | GTX | (1/3) |
| Quercetin | Que | NGTX | NGTX | (3/3) |
| 17beta-estradiol | E2 | NGTX | NGTX | (3/3) |
| Ampicillin trihydrate | AmpC | NGTX | NGTX | (3/3) |
| Cyclosporine A | CsA | NGTX | NGTX | (3/3) |
| 2,3,7,8-tetrachloro dibenzo-p-dioxin | TCDD | NGTX | NGTX | (2/3) |
| | | | 89% | 21/27 |
| Correctness of prediction | | | | (78%) |

**Table 6: Aggregated prediction using 3 different microRNAs.**

| **Compound** | **Abbrevi ation** | **GTX in vivo** | correctness of miRNA prediction | | | Aggregate result | |
|---|---|---|---|---|---|---|---|
| | | | 1262 | 98 | 30c-1 | result | correct |
| Aflatoxin B1 | AFB1 | GTX | (2/3) | (2/3) | (2/3) | GTX | 3/3 |
| Benzo[a]pyere | BaP | GTX | (2/3) | (2/3) | (2/3) | GTX | 3/3 |
| Cisplatin | CisPl | GTX | (2/3) | (3/3) | (2/3) | GTX | 3/3 |
| 8-quinolinol | 8HQ | NGTX | (2/3) | (3/3) | (1/3) | NGTX | 2/3 |
| Quercetin | Que | NGTX | (3/3) | (1/3) | (3/3) | NGTX | 2/3 |
| 17beta-estradiol | E2 | NGTX | (3/3) | (2/3) | (3/3) | NGTX | 3/3 |
| Ampicillin trihydrate | AmpC | NGTX | (2/3) | (3/3) | (3/3) | NGTX | 3/3 |
| Cyclosporine A | CsA | NGTX | (3/3) | (3/3) | (3/3) | NGTX | 3/3 |
| 2,3,7,8-tetrachloro dibenzo-p-dioxin | TCDD | NGTX | (3/3) | (3/3) | (2/3) | NGTX | 3/3 |
| Correctness of prediction | | | 22/27 | 22/27 | 21/27 | 100% | 25/27 |
| | | | (81%) | (81%) | (78%) | | (93%) |

### Example 7: Inclusion of further miRNAs in the analysis improves the robustness of prediction.

When the expression levels of microRNAs 885-3p, 888*, 98, 1262, 1229, 30c-1*, 891b, 302b, 1246, and 502-3p were included in the prediction analysis, the overall results did not further improve, however, the test was found to be more robust. In particular, the use of human microRNAs is preferred when using human samples, such microRNAs are designated hsa-miR-885-3p, hsa-miR-888*, hsa-miR-98, hsa-miR-1262, hsa-miR-1229, hsa-miR-30c-1*, hsa-miR-891b, hsa-miR-302b, hsa-miR-1246, and hsa-miR-502-3p.

MicroRNAs hsa-miR-885-3p, hsa-miR-888*, hsa-miR-1229, 891b, 302b, 1246, and 502-3p on their own did not predict the genotoxicity of the compounds 100% correctly. However, when combined with other miRNAs the combined robustness or accuracy of prediction improved. Table 8 provides some additional information on the microRNAs used.

**Table 7**

| **Compound** | **Abbrevi ation** | **GTX in vivo** | correctness of microRNA prediction | | | | | | | | | | Aggregate result | |
|---|---|---|---|---|---|---|---|---|---|---|---|---|---|---|
| | | | 1262 | 98 | 30c-1 | 885-3p | 888* | 1229 | 891b | 302b | 1246 | 502-3p | result | correct |
| Aflatoxin B1 | AFB1 | GTX | (2/3) | (2/3) | (2/3) | (1/3) | (3/3) | (1/3) | (2/3) | (2/3) | (1/3) | (2/3) | GTX | 17/30 |
| Benzo[a]pyere | BaP | GTX | (2/3) | (2/3) | (2/3) | (2/3) | (2/3) | (3/3) | (3/3) | (2/3) | (3/3) | (2/3) | GTX | 23/30 |
| Cisplatin | CisPl | GTX | (2/3) | (3/3) | (2/3) | (3/3) | (1/3) | (3/3) | (3/3) | (3/3) | (2/3) | (2/3) | GTX | 24/30 |
| 8-quinolinol | 8HQ | NGTX | (2/3) | (3/3) | (1/3) | (1/3) | (3/3) | (3/3) | (3/3) | (1/3) | (0/3) | (2/3) | NGTX | 19/30 |
| Quercetin | Que | NGTX | (3/3) | (1/3) | (3/3) | (1/3) | (1/3) | (1/3) | (2/3) | (1/3) | (2/3) | (1/3) | NGTX | 16/30 |
| 17beta-estradiol | E2 | NGTX | (3/3) | (2/3) | (3/3) | (1/3) | (0/3) | (2/3) | (1/3) | (2/3) | (2/3) | (1/3) | NGTX | 17/30 |
| Ampicillin trihydrate | AmpC | NGTX | (2/3) | (3/3) | (3/3) | (3/3) | (3/3) | (3/3) | (2/3) | (2/3) | (1/3) | (3/3) | NGTX | 25/30 |
| Cyclosporine A | CsA | NGTX | (3/3) | (3/3) | (3/3) | (2/3) | (2/3) | (2/3) | (2/3) | (3/3) | (2/3) | (2/3) | NGTX | 24/30 |
| 2,3,7,8-tetrachloro dibenzo-p-dioxin | TCDD | NGTX | (3/3) | (3/3) | (2/3) | (3/3) | (1/3) | (2/3) | (2/3) | (2/3) | (3/3) | (2/3) | NGTX | 23/30 |
| Correctness of prediction | | | 22/27 | 22/27 | 21/27 | 17/27 | 16/27 | 20/27 | 20/27 | 18/27 | 16/27 | 17/27 | 100% | 188/270 |
| | | | (81%) | (81%) | (78%) | (63%) | (59%) | (74%) | (74%) | (67%) | (59%) | (63%) | | (70%) |

**Table 8: Additional Information (microRNA sequences, MiRBase):**

| **v15 miRBase_ID** | **v18 miRBase_ID** | **Mature_Acc** | **Sequence** | |
|---|---|---|---|---|
| hsa-miR-885-3p | hsa-miR-885-3p | MIMA T0004948 | AGGCAGCGGGGUGUAGUGGAUA | SEQ ID NO: 1 |
| hsa-miR-888* | hsa-miR-888-3p | MIMA T0004917 | GACUGACACCUCUUUGGGUGAA | SEQ ID NO: 2 |
| hsa-miR-98 | hsa-miR-98 | MIMA T0000096 | UGAGGUAGUAAGUUGUAUUGUU | SEQ ID NO: 3 |
| hsa-miR-1262 | hsa-miR-1262 | MIMA T0005914 | AUGGGUGAAUUUGUAGAAGGAU | SEQ ID NO: 4 |
| hsa-miR-1229 | hsa-miR-1229 | MIMA T0005584 | CUCUCACCACUGCCCUCCCACAG | SEQ ID NO: 5 |
| hsa-miR-30c-1* | hsa-miR-30c-1-3p | MIMA T0004674 | CUGGGAGAGGGUUGUUUACUCC | SEQ ID NO: 6 |
| hsa-miR-891b | hsa-miR-891b | MIMA T0004913 | UGCAACUUACCUGAGUCAUUGA | SEQ ID NO: 7 |
| hsa-miR-302b | hsa-miR-302 b-5p | MIMA T0000714 | ACUUUAACAUGGAAGUGCUUUC | SEQ ID NO: 8 |
| hsa-miR-1246 | hsa-miR-1246 | MIMA T0005898 | AAUGGAUUUUUGGAGCAGG | SEQ ID NO: 9 |
| hsa-miR-502-3p | hsa-miR-502-3p | MIMA T0004775 | AAUGCACCUGGGCAAGGAUUCA | SEQ ID NO: 10 |

### Literature incorporated by reference, cited references.

1. Dambach, et al., Toxicol Pathol, 2005. 33(1): p. 17-26.
2. Tsujimura, K., et al., Cancer Sci, 2006. 97(10): p. 1002-10.
3. Kirkland, D., et al., Mutat Res, 2005. 584(1-2): p. 1-256.
4. Chhabra, R.S., Environ Health Perspect, 1979. 33: p. 61-9.
5. Le Fevre, A.C., et al., Mutat Res, 2007. 619(1-2): p. 16-29.
6. Eun, J.W., et al., Toxicology, 2008. 249(2-3): p. 176-83.
7. Aubrecht, J., et al., Toxicol Appl Pharmacol, 1999. 154(3): p. 228-35.
8. Ellinger-Ziegelbauer, H., et al., Toxicol Lett, 2009. 186(1): p. 36-44.
9. Ellinger-Ziegelbauer, H., et al., Mutat Res, 2007.
10. Ellinger-Ziegelbauer, H., et al., Toxicol Sci, 2004. 77(1): p. 19-34.
11. Ellinger-Ziegelbauer, H., et al., Mutat Res, 2005. 575(1-2): p. 61-84.
12. Nioi, P., et al., Chem Biol Interact, 2008. 172(3): p. 206-15.
13. Waterston, R.H., et al., Nature, 2002. 420(6915): p. 520-62.
14. Koike, M., et al., J Radiat Res (Tokyo), 2008.
15. Rogakou, E.P., et al., J Biol Chem, 1998. 273(10): p. 5858-68.
16. Seglen, P.O., Methods Cell Biol, 1976. 13: p. 29-83.
17. Casciano, D.A., Drug Metab Rev, 2000. 32(1): p. 1-13.
18. Mathijs, K., et al., Drug Metab Dispos, 2009.
19. Koebe, H.G., et al., Int J Artif Organs, 1994. 17(2): p. 95-106.
20. Beken, S., et al., Methods Mol Biol, 1998. 107: p. 303-9.
21. Hamer, G., et al., Biol Reprod, 2003. 68(2): p. 628-34.
22. Tong, W., et al., Environ Health Perspect, 2003. 111(15): p. 1819-26.
23. Tong, W., et al., Mutat Res, 2004. 549(1-2): p. 241-53.
24. Irizarry, R.A., et al., Biostatistics, 2003. 4(2): p. 249-64.
25. Affymetrix, Statistical Algorithms Description Document, technical report. 2002.
26. Shi, L., et al., Nat Biotechnol, 2006. 24(9): p. 1151-61.
27. Tibshirani, R., et al., Proc Natl Acad Sci U S A, 2002. 99(10): p. 6567-72.
28. Fernandez-Capetillo, O., et al., DNA Repair (Amst), 2004. 3(8-9): p. 959-67.
29. Rogakou, E.P., et al., J Cell Biol, 1999. 146(5): p. 905-16.
30. Mladenov, E., I. Tsaneva, and B. Anachkova, J Cell Physiol, 2007. 211(2): p. 468-76.
31. Yamamoto, K., et al., Mol Med, 2008.14(3-4): p. 167-74.
32. Zhou, C., et al., Mutat Res, 2006. 604(1-2): p. 8-18.
33. Hoogervorst, E.M., et al., DNA Repair (Amst), 2005. 4(1): p. 3-9.
34. Schrenk, D., et al., Carcinogenesis, 1994. 15(11): p. 2541-6.
35. Jenkins, G.J. and J.M. Parry, Teratog Carcinog Mutagen, 2000. 20(3): p. 107-17.
36. Moller, M.E., et al., Carcinogenesis, 1984. 5(6): p. 797-804.
37. Vu, V.T., et al., Carcinogenesis, 1985. 6(1): p. 45-52.
38. Heflich, R.H. and R.E. Neft, Mutat Res, 1994. 318(2): p. 73-114.
39. Sionov, R.V. and Y. Haupt, Oncogene, 1999. 18(45): p. 6145-57.
40. lida, M., et al., Carcinogenesis, 2005. 26(3): p. 689-99.
41. Uehara, T., et al., Toxicology, 2008. 250(1): p. 15-26.
42. van Delft, J.H., et al., Carcinogenesis, 2004. 25(7): p. 1265-76.

## Claims

1. In vitro method for determining whether a compound is a genotoxic or non-genotoxic compound, said method comprising the steps of:
a. exposing a cell to the compound for a certain amount of time,
b. determining the expression level of at least one microRNA marker in said cell,
c. comparing said expression level with a predetermined reference value and determining whether the expression level of the at least one microRNA marker is below or above the reference value,
d. determining whether the compound is a genotoxic compound or a non-genotoxic compound
wherein the at least one microRNA marker is microRNA 1262.

2. In vitro method according to claim 1 wherein the at least one microRNA is microRNA 1262, microRNA 98 and microRNA 30c-1.

3. In vitro method according to claim 1 wherein the at least one microRNA is microRNA 1262, microRNA 98, microRNA 30c-1, microRNA 885-3p, microRNA 888*, microRNA 1229, microRNA 891b, microRNA302b, microRNA 1246 and microRNA 502-3p.

4. In vitro method according to any of claims 1 - 3 wherein the cell line is HepG2.

5. In vitro method according to any of claims 1 - 4 wherein the amount of time is 12 - 24 hours.

6. In vitro method according to any of claims 1 - 5 wherein method steps a - d are performed at least in triplicate thereby obtaining expression levels at least in triplicate and wherein the determining whether the compound is a genotoxic compound or a non-genotoxic compound is based on the average of such at least in triplicate expression levels.

7. Method according to any one of claims 1 - 6 wherein the reference value is an average value of the level of expression of the at least one microRNA in the cell when the cell is not exposed to the compound.
